(19)

**Europäisches Patentamt**

**European Patent Office**

**Office européen des brevets**

(11) **EP 4 693 302 A1**

(12) **EUROPEAN PATENT APPLICATION**

(43) Date of publication:
**11.02.2026 Bulletin 2026/07**

(21) Application number: **25194642.2**

(22) Date of filing: **07.08.2025**

(51) International Patent Classification (IPC):
**G16C 20/10** *(2019.01)*

(52) Cooperative Patent Classification (CPC):
**G16C 20/10;** G16C 20/70

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: **09.08.2024 KR 20240106756**

(71) Applicant: **Samsung Electronics Co., Ltd**
**Suwon-si, Gyeonggi-do 16677 (KR)**

(72) Inventors:
• **SHIN, Hunsik**
**16678 Suwon-si (KR)**

• **BAE, Jinkyu**
**16678 Suwon-si (KR)**
• **JEONG, Seokyong**
**16678 Suwon-si (KR)**
• **NAM, Sang Ki**
**16678 Suwon-si (KR)**
• **YOO, Suyoung**
**16678 Suwon-si (KR)**
• **JO, Young Hyun**
**16678 Suwon-si (KR)**

(74) Representative: **Arnold & Siedsma**
**Bezuidenhoutseweg 57**
**2594 AC The Hague (NL)**

(54) **METHOD AND DEVICE WITH CHEMICAL REACTION SIMULATION**

(57) A processor-implemented method includes performing, based on an original chemical species database (DB) and an original chemical reaction DB, a low-dimensional simulation based on lower dimensional data and/or determinations than those of a target simulation corresponding to a chemical reaction to be analyzed, the original chemical species DB comprising information corresponding to a chemical species used for the target simulation, and the original chemical reaction DB comprising information corresponding to a chemical reaction used for the target simulation, generating a reduced chemical reaction DB by excluding information corresponding to one or more chemical reactions from the original chemical reaction DB based on a result of the low-dimensional simulation, and performing the target simulation based on the reduced chemical reaction DB and a final chemical species DB.

FIG. 2

EP 4 693 302 A1

**Description**

BACKGROUND

1. Field

The following description relates to a method and device with chemical reaction simulation.

2. Description of the Related Art

A technique for simplifying complex chemical reaction networks to improve simulation efficiency may involve quantifying importance of each chemical species and reaction and selecting key species and reactions when given data on a large number of chemical species and possible chemical reactions occurring therebetween. To this end, there is a method in which an expert with a high level of knowledge of processes and chemical reactions pre-screens candidate chemical species and reactions, and then constructs a simplified set of chemical reactions through analytical techniques such as sensitivity analysis (SA) and pathway analysis (PWA). Alternatively, there is a method to model the given chemical reaction data as a graph network and determine importance of each element based on the connectivity between chemical species and reactions.

Methods utilizing sensitivity analysis and path analysis by experts require prior screening of major chemical species and reactions based on deep prior knowledge of specific processes and chemical reactions, so there is a technical problem in that they cannot be universally applied to various process conditions and chemical reaction systems. On the other hand, methods based on graph networks may be applied to various processes and chemical reaction systems without prior knowledge based on given data, but they have the technical problem of not sufficiently considering that the main chemical reaction mechanism may vary depending on process conditions such as pressure and applied voltage even for the same chemical species and reactions.

To address these issues, effects of changes in process conditions may be considered by performing simulations in advance using chemical species and reaction data and reflecting the additional information obtained from them in graph modeling. However, when computational complexity of the simulation increases due to an increase in the interpretation dimension, it may be very inefficient to perform additional high-dimensional simulations to obtain a simplified chemical reaction network. On the other hand, when all chemical reaction information is directly reflected in graph modeling, an influence of the most critical chemical reactions in the overall mechanism may be relatively underestimated, making it difficult to accurately determine importance of key species and reducing an opportunity to further simplify complex chemical reaction networks. Furthermore, since there is no specific reference for the number of chemical species that can be removed based on the generated importance ranking, there may be large variations in a size of the simplified chemical reaction network and performance of simulating existing chemical reactions depending on a user, which may limit the automation of an entire process to make it more efficient.

SUMMARY

This Summary is provided to introduce a selection of concepts in a simplified form that are further described below in the Detailed Description. This Summary is not intended to identify key features or essential features of the claimed subject matter, nor is it intended to be used as an aid in determining the scope of the claimed subject matter.

In one or more general aspects, a processor-implemented method incudes performing, based on an original chemical species database (DB) and an original chemical reaction DB, a low-dimensional simulation based on lower dimensional data and/or determinations than those of a target simulation corresponding to a chemical reaction to be analyzed, the original chemical species DB comprising information corresponding to a chemical species used for the target simulation, and the original chemical reaction DB comprising information corresponding to a chemical reaction used for the target simulation, generating a reduced chemical reaction DB by excluding information corresponding to one or more chemical reactions from the original chemical reaction DB based on a result of the low-dimensional simulation, and performing the target simulation based on the reduced chemical reaction DB and a final chemical species DB. The method also comprises controlling at least one parameter of a chemical reaction based on the target simulation.

The generating of the reduced chemical reaction DB may include extracting information corresponding to a chemical species density and a reaction rate from the result of the low-dimensional simulation, and determining contribution to generation and/or consumption of chemical species for all chemical reactions associated with the original chemical reaction DB based on the information corresponding to the density and the reaction rate of the chemical species, and generating a reduced chemical reaction DB by excluding information corresponding to one or more chemical reactions selected based on the contribution from the original chemical reaction DB.

The generating of the reduced chemical reaction DB may include determining a first contribution to production and/or consumption of a first chemical species for all chemical reactions of the first chemical species corresponding to the original chemical reaction DB, registering a chemical reaction whose first contribution exceeds a predetermined first threshold among all chemical reactions in the original chemical reaction DB in a white list, and generating the reduced chemical reaction DB by excluding information corresponding to one or more chemical reactions that are not registered in the white list from the original chemical reaction DB.

The generating of the reduced chemical reaction DB may further include determining a second contribution to production and/or consumption of a second chemical species for all chemical reactions of the second chemical species corresponding to the original chemical reaction DB and different from the first chemical species, and registering a chemical reaction having the second contribution that exceeds the first threshold, among all chemical reactions of the original chemical reaction DB, in the white list.

The method may include generating a graph defining the chemical species and the chemical reaction as nodes based on the result of the low-dimensional simulation and the reduced chemical reaction DB, and generating the final chemical species DB by excluding nodes corresponding to one or more chemical species from the graph, wherein the performing of the target simulation may include performing the target simulation based on the final chemical species DB.

The generating of the graph may include forming an edge between a node representing a chemical species and a node representing a chemical reaction based on the reduced chemical reaction DB, depending on whether a chemical reaction is involved, and setting a value of a reaction rate as an edge weight based on a result of the low-dimensional simulation.

The generating of the graph may include generating an undirected species-reaction bipartite graph as a graph using the node, the edge, and the edge weight.

The generating of the final chemical species DB may include determining an importance score for all nodes in the graph, determining an importance ranking for all chemical species by considering the scores for the nodes representing chemical species in the graph, and generating the final chemical species DB based on the importance ranking.

The generating of the final chemical species DB may include determining a reduced set formed of a predetermined number of chemical species selected in order of increasing importance, determining a full set formed of all chemical species represented in the graph, determining a relative error between a result of the low-dimensional simulation performed on the reduced set and a result of the low-dimensional simulation performed on the full set, and generating the reduced set having the relative error less than or equal to a predetermined second threshold as the final chemical species DB.

The relative error may be determined according to the Equation $\mathrm{RE} = \left(\frac{n_R - n_F}{n_F}\right) \times 100$, wherein RE indicates the relative error, $n_R$ indicates a number density of the species of interest based on the reduced set, and $n_F$ indicates a number density of the species of interest based on the full set.

The target simulation may include a low-temperature plasma simulation for semiconductor process analysis.

In one or more general aspects, an electronic device includes one or more processors comprising processing circuitry, and memory comprising one or more storage media storing instructions that, when executed individually or collectively by the one or more processors, cause the electronic device to perform, based on an original chemical species database (DB) and an original chemical reaction DB, a low-dimensional simulation based on lower dimensional data and/or determinations than those of a target simulation corresponding to a chemical reaction to be analyzed, the original chemical species DB comprising information corresponding to a chemical species used for the target simulation, and the original chemical reaction DB comprising information corresponding to a chemical reaction used for the target simulation, generate a reduced chemical reaction DB by excluding information corresponding to one or more chemical reactions from the original chemical reaction DB based on a result of the low-dimensional simulation, and perform the target simulation based on the reduced chemical reaction DB.

For the generating of the reduced chemical reaction DB, the execution of the instructions may cause the electronic device to extract information corresponding to a chemical species density and a reaction rate from the result of the low-dimensional simulation, and determine contribution to generation and/or consumption of chemical species for all chemical reactions associated with the original chemical reaction DB based on the information corresponding to the density and the reaction rate of the chemical species, and generate a reduced chemical reaction DB by excluding information corresponding to one or more chemical reactions selected based on the contribution from the original chemical reaction DB.

For the generating of the reduced chemical reaction DB, the execution of the instructions may cause the electronic device to determine a first contribution to production and/or consumption of a first chemical species for all chemical reactions of the first chemical species corresponding to the original chemical reaction DB, register a chemical reaction whose first contribution exceeds a predetermined first threshold among all chemical reactions in the original chemical reaction DB in a white list, and generate the reduced chemical reaction DB by excluding information corresponding to one or more chemical reactions that are not registered in the white list from the original chemical reaction DB.

In one or more general aspects, a processor-implemented method includes performing, based on an original chemical species database (DB) and an original chemical reaction DB, a low-dimensional simulation based on lower dimensional data and/or determinations than those of a target simulation corresponding to a chemical reaction to be analyzed, the original chemical species DB comprising information corresponding to a chemical species used for the target simulation, and the original chemical reaction DB comprising information corresponding to a chemical reaction used for the target simulation, generating a graph defining the chemical species and the chemical reaction as nodes based on a result of the low-dimensional simulation, generating a final chemical species DB by excluding nodes corresponding to one or more chemical species from the graph, and performing the target simulation based on the final chemical species DB.

The generating of the graph may include forming an edge between a node representing a chemical species and a node representing a chemical reaction depending on whether a chemical reaction is involved, and setting a value of a reaction rate as an edge weight based on a result of the low-dimensional simulation.

The generating of the graph may include generating an undirected species-reaction bipartite graph as a graph using the node, the edge, and the edge weight.

The generating of the final chemical species DB may include determining an importance score for all nodes in the graph, determining an importance ranking for all chemical species by considering the scores for the nodes representing chemical species in the graph, and generating the final chemical species DB based on the importance ranking.

The generating of the final chemical species DB may include determining a reduced set formed of a predetermined number of chemical species selected in order of increasing importance, determining a full set formed of all chemical species represented in the graph. determining a relative error between a result of the low-dimensional simulation performed on the reduced set and a result of the low-dimensional simulation performed on the entire set, and generating the reduced set having the relative error less than or equal to a predetermined second threshold as the final chemical species DB.

The relative error may be determined according to the Equation $RE = \left( \frac{n_R - n_F}{n_F} \right) \times 100$, wherein RE indicates the relative error, $n_R$ indicates a number density of the species of interest based on the reduced set, and $n_F$ indicates a number density of the species of interest based on the full set.

The method of the present application can be applied to control at least one parameter of a chemical reaction based on the target simulation. The chemical reaction may be a manufacturing process. For example, the method controls at least one parameter of a process of manufacturing semiconductor.

Other features and aspects will be apparent from the following detailed description, the drawings, and the claims.

BRIEF DESCRIPTION OF THE DRAWINGS

FIG. 1 illustrates a chemical reaction simulation device according to one or more embodiments.
FIG. 2 to FIG. 6B illustrate a view for describing specific operations of a chemical reaction simulation according to one or more embodiments.
FIG. 7 illustrates a chemical reaction simulation method according to one or more embodiments.
FIG. 8 to FIG. 11 illustrate results of chemical reaction simulations according to one or more embodiments.
FIG. 12 illustrates a chemical reaction simulation method according to one or more embodiments.
FIG. 13 illustrates a chemical reaction simulation method according to one or more embodiments.
FIG. 14 illustrate a view for describing specific operations of a chemical reaction simulation according to one or more embodiments.
FIG. 15 illustrates a view for describing a computing device according to one or more embodiments.

Throughout the drawings and the detailed description, unless otherwise described or provided, the same drawing reference numerals may be understood to refer to the same elements, features, and structures. The drawings may not be to scale, and the relative size, proportions, and depiction of elements in the drawings may be exaggerated for clarity, illustration, and convenience.

DETAILED DESCRIPTION

The following detailed description is provided to assist the reader in gaining a comprehensive understanding of the methods, apparatuses, and/or systems described herein. However, various changes, modifications, and equivalents of the methods, apparatuses, and/or systems described herein will be apparent after an understanding of the disclosure of this application. For example, the sequences within and/or of operations described herein are merely examples, and are not limited to those set forth herein, but may be changed as will be apparent after an understanding of the disclosure of this application, except for sequences within and/or of operations necessarily occurring in a certain order. As another example, the sequences of and/or within operations may be performed in parallel, except for at least a portion of sequences of and/or within operations necessarily occurring in an order, e.g., a certain order. Also, descriptions of features that are known after an understanding of the disclosure of this application may be omitted for increased clarity and conciseness.

The terminology used herein is for describing various examples only and is not to be used to limit the disclosure. The articles "a," "an," and "the" are intended to include the plural forms as well, unless the context clearly indicates otherwise. As non-limiting examples, terms "comprise" or "comprises," "include" or "includes," and "have" or "has" specify the presence of stated features, numbers, operations, members, elements, and/or combinations thereof, but do not preclude the presence or addition of one or more other features, numbers, operations, members, elements, and/or combinations thereof, or the alternate presence of an alternative stated features, numbers, operations, members, elements, and/or combinations thereof. Additionally, while one embodiment may set forth such terms "comprise" or "comprises," "include" or "includes," and "have" or "has" specify the presence of stated features, numbers, operations, members, elements, and/or combinations thereof, other embodiments may exist where one or more of the stated features, numbers, operations, members, elements, and/or combinations thereof are not present.

Although terms such as "first," "second," and "third", or A, B, (a), (b), and the like may be used herein to describe various members, components, regions, layers, or sections, these members, components, regions, layers, or sections are not to be limited by these terms. Each of these terminologies is not used to define an essence, order, or sequence of corresponding members, components, regions, layers, or sections, for example, but used merely to distinguish the corresponding members, components, regions, layers, or sections from other members, components, regions, layers, or sections. Thus, a first member, component, region, layer, or section referred to in the examples described herein may also be referred to as a second member, component, region, layer, or section without departing from the teachings of the examples.

Throughout the specification, when a component or element is described as being "on", "connected to," "coupled to," or "joined to" another component, element, or layer it may be directly (e.g., in contact with the other component, element, or layer) "on", "connected to," "coupled to," or "joined to" the other component, element, or layer or there may reasonably be one or more other components, elements, layers intervening therebetween. When a component, element, or layer is described as being "directly on", "directly connected to," "directly coupled to," or "directly joined" to another component, element, or layer there can be no other components, elements, or layers intervening therebetween. Likewise, expressions, for example, "between" and "immediately between" and "adjacent to" and "immediately adjacent to" may also be construed as described in the foregoing.

As used herein, the term "and/or" includes any one and any combination of any two or more of the associated listed items. The phrases "at least one of A, B, and C", "at least one of A, B, or C", and the like are intended to have disjunctive meanings, and these phrases "at least one of A, B, and C", "at least one of A, B, or C" (e.g., each phrase may include any one of the respective items alone, all of the items listed together, and all possible combinations thereof), and the like also include examples where there may be one or more of each of A, B, and/or C (e.g., any combination of one or more of each of A, B, and C), unless the corresponding description and embodiment necessitates such listings (e.g., "at least one of A, B, and C") to be interpreted to have a conjunctive meaning.

Unless otherwise defined, all terms, including technical and scientific terms, used herein have the same meaning as commonly understood by one of ordinary skill in the art to which this disclosure pertains and specifically in the context on an understanding of the disclosure of the present application. Terms, such as those defined in commonly used dictionaries, are to be interpreted as having a meaning that is consistent with their meaning in the context of the relevant art and specifically in the context of the disclosure of the present application, and are not to be interpreted in an idealized or overly formal sense unless expressly so defined herein.

The features described herein may be embodied in different forms, and are not to be construed as being limited to the examples described herein. Rather, the examples described herein have been provided merely to illustrate some of the many possible ways of implementing the methods, apparatuses, and/or systems described herein that will be apparent after an understanding of the disclosure of this application. The use of the term "may" herein with respect to an example or embodiment (e.g., as to what an example or embodiment may include or implement) means that at least one example or embodiment exists where such a feature is included or implemented, while all examples are not limited thereto. The use of the terms "example", "embodiment", and "example embodiment" herein have a same meaning (e.g., the phrasing 'in an or one example' has a same meaning as 'in an or one embodiment" and 'in an or one example embodiment'), and "one or more examples" has a same meaning as "one or more embodiments" and "one or more example embodiments". Still further, each of multiple or all separately described an/one "example", "embodiment", "example embodiment", as well as "examples", "embodiments", "example embodiments", herein may be included, in combination, in a same embodiment in any combination.

Terms such as "part," "portion," "module," etc., described in the specification may indicate hardware configured to process at least one function or operation described in the specification, and this may be implemented by hardware or a circuit, or a combination of hardware or a circuit implementing software. Furthermore, one or more of the components or functions of the chemical reaction simulation methods and devices according to the embodiments described below may be implemented as hardware implementing a program or software, and the program or software may be stored in a computer-readable medium.

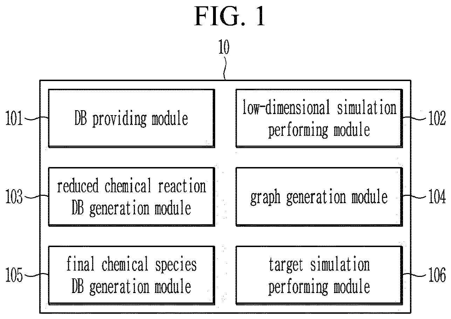

FIG. 1 illustrates a chemical reaction simulation device according to one or more embodiments.

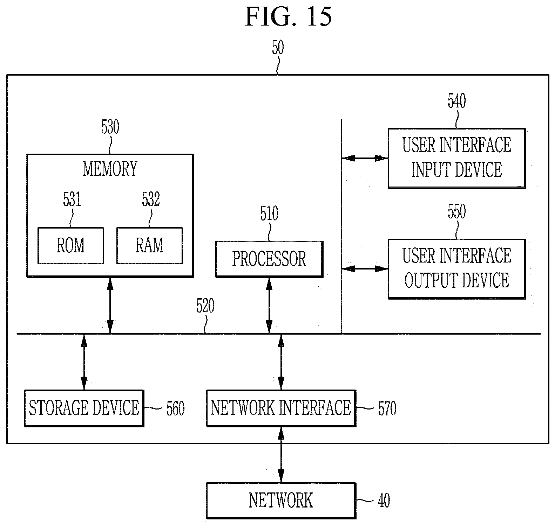

Referring to FIG. 1, a chemical reaction simulation device 10 according to one or more embodiments may execute program codes or instructions loaded in one or more memory devices through one or more processors. For example, the chemical reaction simulation device 10 may be implemented as a computing device 50 as described below with reference to FIG. 15. In this case, one or more processors may correspond to a processor 510 of the computing device 50, and one or more memory devices may correspond to a memory 530 of the computing device 50. The program codes or instructions may be executed by the one or more processors to perform chemical reaction simulations.

The chemical reaction simulation device 10 of one or more embodiments may simplify a chemical reaction network used in a chemical reaction simulation, thereby increasing stability of the simulation and shortening an analysis time thereof. For example, the chemical reaction simulation may include a low-temperature plasma simulation for semiconductor process analysis, and the chemical reaction simulation device 10 of one or more embodiments may significantly shorten analysis times of 2D and 3D plasma simulations for accurately analyzing semiconductor processes and facilities, thereby enabling more effective analysis of process issues involving complex chemical reactions. Furthermore, an increase in simulation efficiency provided by the chemical reaction simulation device 10 of one or more embodiments may significantly reduce a time and cost required for development and optimization of semiconductor processes, and may also predict, resolve, and prevent problems occurring in actual processes and facilities. Of course, the chemical reaction simulation device 10 may be applied not only to plasma including charged particles such as electrons or ions, but also to determine key factors in chemical reaction networks of general gases, liquids, and solids, and to simplify complex reaction paths. Accordingly, in addition to a semiconductor manufacturing process, the chemical reaction simulation device 10 of one or more embodiments may simplify a complex chemical reaction to increase stability and efficiency of simulation and analysis, thereby shortening a development period of products including a corresponding chemical process and optimizing the process. The method of the present application can be applied to control at least one parameter of a chemical reaction (such as a manufacturing process) including plasma, and/or general gases, and/or liquids, and/or solids.

For example, the chemical reaction simulation device 10 of one or more embodiments may ameliorate problems that cannot be universally applied due to insufficient information related to various process conditions and chemical reaction systems in a method of selecting important species and reactions through domain knowledge and graph modeling, and/or problems that require high computational complexity due to high interpretation dimension in a method of utilizing sensitivity analysis and pathway analysis by experts, and obtains information related to various process conditions and reactions through a low-dimensional simulation, and may set a reference for reducing chemical reactions and chemical species to reduce complexity. Furthermore, the chemical reaction simulation device 10 of one or more embodiments may significantly shorten a time required for a high-dimensional simulation by extracting chemical species and reactions essential for simulation analysis and reducing simulation input, thereby ensuring stability and providing optimization of system development.

For example, the chemical reaction simulation device 10 may include a database (DB) providing module 101, a low-dimensional simulation performing module 102, a reduced chemical reaction DB generation module 103, a graph generation module 104, a final chemical species DB generation module 105, and a target simulation performing module 106.

The DB providing module 101 may receive an original chemical species DB including information related to chemical species used for a target simulation related to a chemical reaction to be analyzed. Chemical species may include many chemical forms, such as atoms, molecules, ions, radicals, and complexes, that can undergo chemical reactions or physical changes. For example, the chemical species may have various forms, such as atoms (e.g., helium (He) or oxygen (O)), molecules (e.g., carbon dioxide ($CO_2$) or water ($H_2O$)), ions (e.g., sodium ion ($Na^+$), chloride ion (Cl)), radicals (e.g., hydroxyl radical (OH·)), and complexes (e.g., specific reaction intermediates or catalytic complexes). The chemical species may act as reactants and/or products in chemical reactions, and may change into different forms depending on conditions and/or environments of the reaction. For example, as a chemical reaction progresses, atoms may combine to form new molecules, and/or existing molecules may break apart into individual atoms or ions. Accordingly, the chemical species may refer to all chemical components observed within a particular reaction or system, and may be of great importance in chemical analysis or simulation. In one or more embodiments, an original chemical species DB may store and manage names of all chemical species utilized in a target simulation.

Meanwhile, the DB providing module 101 may receive an original chemical reaction DB including information related to a chemical reaction used for a target simulation. The chemical reaction may indicate a process in which one or more chemical species interact to be converted into a new chemical species. The chemical reaction may occur when existing chemical bonds are broken and new bonds are formed, and in this process, properties or a state of a substance may change. Initial chemical species that participate when the chemical reaction begins may be called reactants, and newly created chemical species after the reaction is completed may be called products. For example, reactants may interact with each other during a reaction process, may undergo changes, and may be transformed into new substances, and the products may have different chemical structures and properties from chemical structures and properties of the reactants and may be obtained as a result of the reaction. In one or more embodiments, the original chemical reaction DB may store and manage information to be used for chemical reaction determinations, such as physical information and reaction coefficients of all chemical species utilized in the target simulation.

The low-dimensional simulation performing module 102 may perform a low-dimensional simulation based on data and/or determinations of a lower dimension than the target simulation for the original chemical species DB and the original chemical reaction DB described above. Herein, the low-dimensional simulation may indicate a simulation that may produce simulation results relatively much faster than the target simulation by using lower-dimensional data and/or determinations than those of the target simulation. In one or more embodiments, the low-dimensional simulation may be performed by taking as input not only the chemical species DB and the chemical reaction DB, but also physical and numerical information required for driving, e.g. for driving the simulation, and may generate various properties related to considered chemical species and reactions as result files.

The term simulation, as used herein, may refer to a simulation that includes a numerical model for simulating a behavior of a chemical reaction, and such a simulation may predict chemical reaction rates and amounts (or number density) of chemical species produced and consumed over time, given specific variables such as temperature, pressure, etc., and chemical species and reactions between chemical species. A low-temperature plasma simulation for semiconductor process analysis is one type of simulation that includes chemical reaction determinations, and in addition, various simulations that perform chemical reaction determinations such as combustion reactions, gas-surface reactions, catalytic reactions, and biochemical reactions may be included in a term simulation used in this specification. The method of the present application can be applied to control at least one parameter of a chemical reaction, such as a chemical reaction in manufacturing and/or testing a process. The chemical reaction may be at least the one of the following reactions: plasma reaction (such as a low-temperature plasma reaction), combustion reactions, gas-surface reactions, catalytic reactions, and biochemical reactions.

In the case of low-temperature plasma simulation for semiconductor process analysis, a chemical reaction model may be determined according to process variables such as a process gas, a flow rate, a temperature, an applied voltage, a chamber shape, and physical properties to predict chemical reaction rates such as ionization, dissociation, and excitation of the process gas, and a spatiotemporal distribution of a density of chemical species such as newly generated electrons, ions, and highly reactive radicals may be predicted.

Simulations may be classified into low-dimensional simulations and high-dimensional simulations as a relative sense, depending on complexity of the model that simulates an actual phenomenon, and space-time dimensions considered in the simulation, resolution, diversity and accuracy of the physical model, etc. may be used as a reference for this classification. In this specification, a high-dimensional simulation as opposed to the low-dimensional simulation may also be referred to as a target simulation, and distinction between the low-dimensional simulation and the target simulation may be made based on whether more detailed physical phenomena are present such as spatial distribution and transport of particles by electromagnetic fields. For example, a global model corresponding to a low-dimensional plasma simulation may determine production and/or consumption of chemical species and reaction rates by volume-averaging them without considering distribution of density over space, whereas a target simulation corresponding to a high-dimensional plasma simulation may predict density of chemical species that varies with positions in time and space by considering transport of particles by electromagnetic fields and fluids.

The reduced chemical reaction DB generation module 103 may generate a reduced chemical reaction DB by excluding information related to one or more chemical reactions from an original chemical reaction DB based on results of the low-dimensional simulation. Herein, the reduced chemical reaction DB may represent the original chemical reaction DB excluding reactions with low contribution influencing the production and/or consumption of chemical species.

In one or more embodiments, the reduced chemical reaction DB generation module 103 may extract information related to density and a reaction speed of the chemical species from the results of the low-dimensional simulation. The chemical species may be created or consumed by a reaction, and the density of corresponding chemical species may be determined accordingly. Meanwhile, the reaction speed may be determined by a product of the densities of chemical species participating in the reaction and a product of a reaction constant. The reduced chemical reaction DB generation module 103 may determine contribution to generation and/or consumption of chemical species based on information related to the density and the reaction rate of the chemical species. Furthermore, the reduced chemical reaction DB generation module 103 may generate a reduced chemical reaction DB by excluding information related to one or more chemical reactions selected based on the contribution from the original chemical reaction DB.

In a typical chemical reaction system, a number of chemical reactions that can occur between chemical species may be several to ten times greater than a number of chemical species that constitutes the system. Accordingly, in the case of a bipartite graph that considers both chemical species and chemical reactions as nodes as described below with respect to the graph generation module 104, one chemical species may be connected to innumerable chemical reactions. Meanwhile, as described below with respect to the final chemical species DB generation module 105, importance determined for each node may be affected by a number of adjacent nodes, so when there is a chemical species linked to a large number of unimportant chemical reactions, the typical chemical reaction system may evaluate importance of the chemical species too high, thereby distorting relative importance.

To reduce such distortion of the typical chemical reaction system, the reduced chemical reaction DB generation module of one or more embodiments may obtain (e.g., determine and/or generate) additional information (e.g., reaction rates, etc.) by utilizing low-dimensional simulations that require relatively little interpretation time, and may perform screening to selectively remove chemical reactions that contribute very little to the production and/or consumption of each chemical species. The graph generation module 104 may construct a binary graph based on the chemical reactions and chemical species selected through this process, and an edge weight between the chemical species and reactions may reflect a reaction rate value obtained through a low-dimensional simulation. By utilizing the low-dimensional simulation in this way, the chemical reaction simulation device 10 of one or more embodiments may reduce unnecessary nodes and may reflect the characteristics of chemical systems (e.g., plasma chemical systems) that change depending on various process conditions (such as temperature and pressure) in a graph network.

For example, the reduced chemical reaction DB generation module 103 of one or more embodiments may determine reaction formulas that can be removed without any effect on the density of each species in a substantially steady state among reaction formulas, thereby removing them from the original chemical reaction DB. Herein, the steady state may indicate a state in which density of each species no longer changes after a sufficient time has passed in a space where a chemical reaction is taking place. A final density in the steady state may be predicted through a simulation.

For example, in Reaction 1, where a chemical species A and a chemical species B react to form a chemical species C, a rate or reaction rate $r_1$ for Reaction 1 may be determined by $k_1[A][B]$. Herein, [A] may indicate a density of the chemical species A, [B] may indicate a density of the chemical species B, and $k_1$ may indicate a reaction constant for Reaction 1. Depending on the reaction rate $r_1$, the densities of the chemical species A and B may decrease and the density of the chemical species C may increase. Furthermore, a change in density [j] of any chemical species j may be determined by a rate at which j is produced and consumed in all reactions. When a number of the chemical species j produced and consumed by reaction i is $\gamma_{i,j}$, the change in the density [j] over time may be as follows, for example:

$$d[j]/dt = \sum_i \gamma_{i,j} \, r_i$$

Herein, $r_i$ indicates a reaction rate of Reaction i.

When a steady state is reached, no species will change any more, and a change rate for each species will become 0. Accordingly, the list of the density [j] for which all of the following equations hold true for all the species j may be a density to be obtained.

Change in density over time when steady state is reached:

$$0 = \sum_i \gamma_{i,j} \, r_i$$

Herein, $r_i$ indicates a product of densities of reactants of Reaction i and a reaction constant.

When any of the values inside $\Sigma$ added in the equation is relatively very small compared to the other values, even when the generation and/or consumption by the equation are not considered, the very small value may not affect the final density [j]. For example, for reactions with low contributions to production and/or consumption, the final result may not be affected even when determinations are not performed for the reactions with low contributions. Using this, contribution of each reaction to production and/or consumption for each chemical species may be determined, and reaction equations having contribution that does not exceed a certain value for any chemical species (e.g., the reactions with low contributions) may be eliminated.

For example, when it is assumed that the chemical species A is consumed one by one by Reactions 1, 2, and 3, and produced one by one by Reactions 4, 5, and 6, then a change in the chemical species A will be as follows, for example:

$$d[A]/dt = - (r_1 + r_2 + r_3) + r_4 + r_5 + r_6$$

Assuming equilibrium here, it may be expressed as follows, for example:

$$r_1 + r_2 + r_3 = r_4 + r_5 + r_6 \equiv R_A$$

For example, total production and total consumption may be the same, and this may be defined as $R_A$. In this case, when both sides are divided by $R_A$, it will be as follows, for example:

$$r_1/R_A + r_2/R_A + r_3/R_A = r_4/R_A + r_5/R_A + r_6/R_A = 1$$

Herein, a sum of each side is 1, and a size of each term has a value between 0 and 1. The size of each term may be considered as a contribution of each reaction to A. For example, $r_3/R_A$ may be considered as a contribution of Reaction 3 to A. When it is assumed that the magnitudes of $r_3/R_A$ and $r_6/R_A$ are less than, e.g., 0.001, then contributions of Reactions 3 and 6 to A may be interpreted as insignificant, and a following approximate equation may be established, for example:

$$r_1 + r_2 \approx r_4 + r_5 \approx R_A$$

When the equation is satisfied, it may be seen that d[A]/dt is still close to 0. For example, even when reaction coefficients of Reactions 3 and 6 are 0 or Reactions 3 and 6 are not considered, an equilibrium state may not be affected. In this way, for all reactions, reactions may be selected that do not affect the balanced equation.

However, Reactions 3 and 6 may not be eliminated directly. This is because Reactions 3 and 6 may not affect the equilibrium of A, but contributions of other chemical species other than A may not be close to 0. Accordingly, reactions (for example, reactions that have a very low contribution to all species) other than reactions that have a high contribution to all chemical species may be finally selected and eliminated at least once. Accordingly, the chemical reaction simulation device 10 of one or more embodiments may improve simulation speed and stability by removing unnecessary reactions from the simulation.

For example, the reduced chemical reaction DB generation module 103 may determine a first contribution to production and/or consumption of the first chemical species for all chemical reactions related to the first chemical species related to the original chemical reaction DB, and may register a chemical reaction whose first contribution exceeds a predetermined first threshold (e.g., 0.01), among all chemical reactions in the original chemical reaction DB, in a white list. Furthermore, the reduced chemical reaction DB generation module 103 may determine a second contribution to production and/or consumption of a second chemical species for all chemical reactions of the second chemical species related to the original chemical reaction DB and different from the first chemical species, and may register a chemical reaction having the second contribution that exceeds the first threshold, among all chemical reactions of the original chemical reaction DB, in the white list. In response to this process being repeatedly performed for all chemical species associated with the original chemical reaction DB, the reduced chemical reaction DB generation module 103 may generate a reduced chemical reaction DB by excluding information related to one or more chemical reactions that are not registered in the white list from the original chemical reaction DB.

The graph generation module 104 may generate a graph defining chemical species and chemical reactions as nodes based on results of a low-dimensional simulation and a reduced chemical reaction DB. For example, the graph generation module 104 may form an edge between a node representing a chemical species and a node representing a chemical reaction based on the reduced chemical reaction DB, depending on whether a chemical reaction is involved, and may set a value of a reaction rate as an edge weight based on a low-dimensional simulation result. In one or more embodiments, the graph generation module 104 may generate an undirected species-reaction bipartite graph as a graph using nodes, edges, and edge weights, such that edges are formed only between a node representing a chemical species and a node representing a chemical reaction, and no edges may be formed between the nodes representing the chemical species and between the nodes representing the chemical reactions. Furthermore, the graph may be generated as an undirected graph, so edges have no direction, and thus the graph may indicate that a relationship between the chemical species and the chemical reaction may be reciprocal or bidirectional.

The final chemical species DB generation module 105 may generate a final chemical species DB by excluding nodes related to one or more chemical species from the graph. For example, the final chemical species DB generation module 105 may determine importance as a score (also called the importance score in this application) for all nodes in the graph. In one or more embodiments, a score for each node may be computed according to Equation 1 below, for example.

Equation 1:

$$P(v) = (1 - d) + d * \sum_{u \in |E(v)|} \frac{1}{w(u, v)} * P(u)$$

In Equation 1, P(v) may indicate a score of a node v, d may indicate a damping factor, E(v) may indicate a neighboring node of the node v, w(u, v) may indicate an edge weight for an edge formed between a node u and the node v, and P(u) may indicate a score of the node u. For example, the score P(v) may be higher the more nodes the node v is connected to and the more highly connected nodes the node v is connected to. The scores for all nodes may be computed recursively until convergence occurs.

The final chemical species DB generation module 105 may produce an importance ranking for all chemical species by considering the scores for nodes representing chemical species in the graph. For example, although the score itself may be determined up to the node representing the chemical reaction, in order to determine the importance of the species in a graph formed of chemical species and reaction formulas, the importance ranking may be determined without considering the score for the node representing the chemical reaction.

The final chemical species DB generation module 105 may generate the final chemical species DB based on the importance ranking. Reducing the number of chemical species may improve an interpretation efficiency of the simulation, but may decrease an interpretation consistency of the simulation. Accordingly, to maintain or increase the interpretation consistency of the simulation, the chemical reaction simulation device 10 of one or more embodiments may use a reference to appropriately reduce the number of chemical species according to the importance ranking. The final chemical species DB generation module 105 may define a reduced set formed of a predetermined number of chemical species selected in order of increasing importance, and may define a full set formed of all chemical species represented in the graph. In response to defining the reduced set and defining the full set, the final chemical species DB generation module 105 may determine a relative error between a result of the low-dimensional simulation performed on the reduced set and a result of the low-dimensional simulation performed on an entire set (e.g., the full set), and may generate the reduced set having the relative error less than or equal to a predetermined second threshold as the final chemical species DB. For example, by using low-dimensional simulations, a maximum number of chemical species may be derived for which the relative error compared to a interpretation result when considering all chemical species remains below a second threshold (e.g., 1%).

In one or more embodiments, the relative error may be computed according to the following Equation 2, for example.

$$\text{Equation 2:}$$

$$\text{RE} = \left(\frac{n_R - n_F}{n_F}\right) \times 100$$

In Equation 2, RE may indicate the relative error (e.g., unit: %), $n_R$ may indicate a number density of the species of interest based on the reduced set, and $n_F$ may indicate a number density of the species of interest based on the full set.

The target simulation execution module 106 may perform target simulation based on the reduced chemical reaction DB and the final chemical species DB. For example, the target simulation execution module 106 may perform more accurate simulation determinations based on high-dimensional data and determinations than that of a low-dimensional simulation of a target phenomenon to be simulated.

In one or more embodiments, the target simulation may include a low temperature plasma simulation for semiconductor process analysis. The low temperature plasma simulation for semiconductor process analysis, which is a simulation that numerically simulates and analyzes a physical and chemical behavior of low temperature plasma used in a semiconductor manufacturing process, may be used for design and optimization of a semiconductor process, problem solving, and prediction. The target simulation execution module 106 may analyze a behavior of particles in the plasma and predict a plasma characteristic based on the reduced chemical reaction DB and the final chemical species DB, and/or may model the chemical reactions occurring in the plasma, and may generate and destruct chemical species occurring under a certain process condition and determine a reaction rate. Furthermore, the target simulation execution module 106 of one or more embodiments may find (e.g., determine) an optimal process condition by adjusting process variables based on the reduced chemical reaction DB and the final chemical species DB, and/or predict problems that may occur during the process in advance.

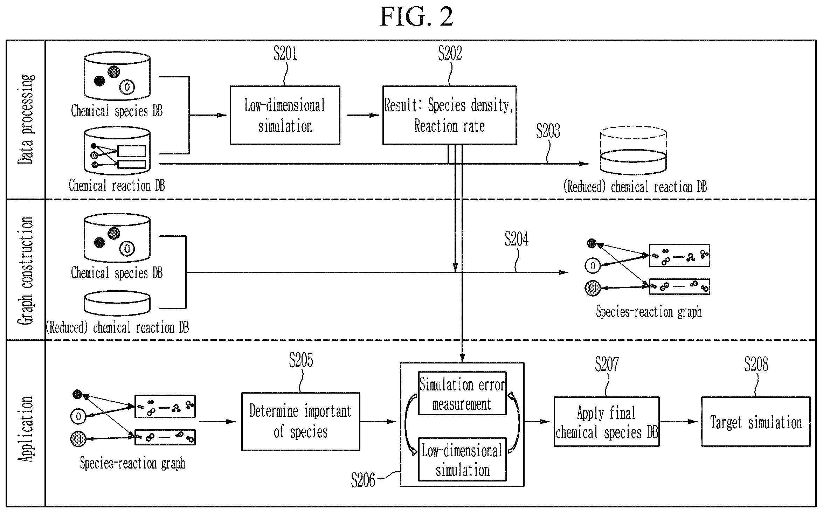

FIG. 2 to FIG. 6B illustrate a view for describing specific operations of a chemical reaction simulation according to one or more embodiments. Operations S201 to S208 of FIG. 2 may be performed in the sequence and manner as illustrated in FIG. 2. However, one or more of the operations may be performed in a different order, one or more of the operations may be omitted, two or more of the operations may be performed in parallel or simultaneously, and/or other operations may be additionally performed without departing from the scope of the described embodiments.

Referring to FIG. 2, a chemical reaction simulation according to one or more embodiments may be implemented to largely include a data processing operation, a graph construction operation, and an application operation.

In the data processing operation, a chemical species DB and a chemical reaction DB may be prepared, each of which may correspond to the original chemical species DB and the original chemical reaction DB described above with respect to FIG. 1. The chemical species DB may be implemented as a DB file including names of all chemical species used in the target simulation, and the chemical reaction DB may be implemented as a DB file including information to be used for determining chemical reactions, such as physical information and reaction coefficients of all chemical species used in the target simulation.

For example, in operation S201, a low-dimensional simulation may be performed based on the chemical species DB and the chemical reaction DB, and the low-dimensional simulation may be implemented as a tool that may obtain results relatively quickly using lower-dimensional data and/or determinations than the target simulation, and various properties related to chemical species and reactions may be determined by inputting physical and numerical information to the low-dimensional simulation. In operation S202, a result of the low-dimensional simulation performed in operation S201 may be obtained (e.g., determined or generated) as a data file in which a density for each chemical species and reaction rate information are extracted from a result file generated from the low-dimensional simulation. In operation S203, the reduced chemical reaction DB may be generated by excluding reactions with low contribution that affect the production and/or consumption of chemical species in the chemical reaction DB, based on the result of the low-dimensional simulation obtained in operation S202.

In the graph construction operation, e.g., operation S204, an undirected chemical species reaction bipartite graph may be generated from a low-dimensional simulation result (e.g., the result of the low-dimensional simulation obtained in operation S202) and information of the reduced chemical reaction DB. In this case, nodes in the graph may represent individual chemical reactions and chemical species, and edges may indicate whether they participate in a chemical reaction, and the edges may connect reactant and product chemical species for individual chemical reactions. Values of reaction rates from low-dimensional simulation results may be used as edge weights.

In the application operation, importance of the chemical species may be determined in operation S205, and a reduced set having a relative error of, e.g., less than 1% may be selected as the final chemical species DB in operation S207 by measuring a relative error between result of a low-dimensional simulation performed on a reduced set of chemical species selected in order of increasing importance in operation S206 and a result of a low-dimensional simulation performed on an entire set of all chemical species represented in the graph generated in operation S204. In operation S208, the target simulation may be performed based on the reduced chemical reaction DB and the final chemical species DB. Herein, the target simulation of one or more embodiments may be implemented as a simulation tool that determines the target phenomenon to be simulated more accurately from higher-dimensional data and determinations than those of the low-dimensional simulation.

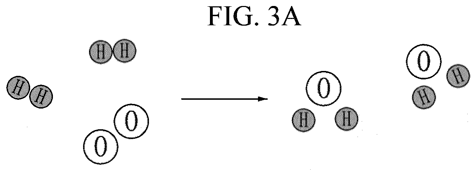

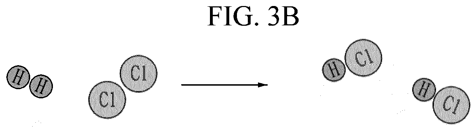

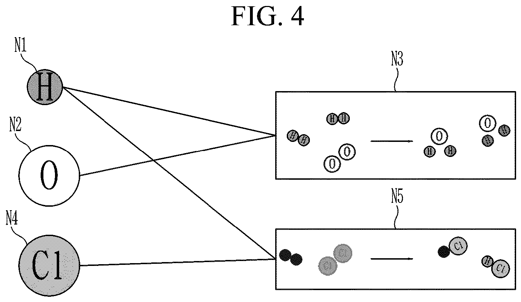

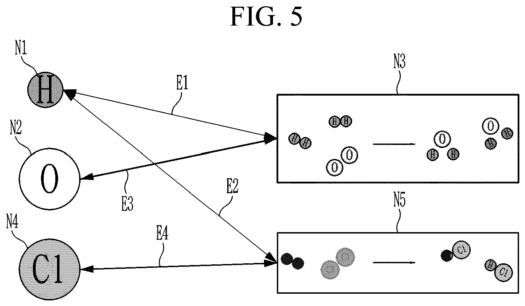

Referring to FIGS. 3A and 3B, FIG. 3A shows a chemical reaction formula $2H_2 + O_2 \rightarrow 2H_2O$, and FIG. 3B shows a chemical reaction formula $H_2 + Cl_2 \rightarrow 2HCl$. Referring to FIG. 4, the chemical reactions of FIGS. 3A and 3B are represented graphically. Herein, nodes N1, N2, and N4 may be defined as nodes representing chemical species, and nodes N3 (e.g., corresponding to FIG. 3A) and N5 (e.g., corresponding to FIG. 3B) may be defined as nodes representing chemical reactions. Next, referring to FIG. 5, it may be seen that an edge E1 is formed between a node N1 and a node N3, an edge E2 is formed between the node N1 and a node N5, an edge E3 is formed between a node N2 and the node N3, and an edge E4 is formed between a node N4 and the node N5, thereby forming an undirected chemical species reaction bipartite graph. In FIG. 5, sizes of nodes N1, N2, and N4 may be different, and for example, it may be seen that the nodes N2 and N4 have a higher score indicating importance than that of the node N1 (e.g., as indicated by the nodes N2 and N4 being larger in size than the node N1), and a thickness of the edge E3 is thickest, indicating that an edge weight of the edge E3 is the largest (e.g., larger than edge weights of the edges E1, E2, and E4), and that a connection strength between the node N2 and the node N3 is strongest. It may be seen that the edge E4 is thinner than edge E3 but thicker than the edges E1 and E2, indicating that an edge weight of the edge E4 is smaller than that of the edge E3 and larger than that of the edges E1 and E2. Furthermore, it may be seen that a connection strength between the node N4 and the node N5 is smaller than a connection strength between the node N2 and the node N3, but larger than a connection strength between the node N1 and the nodes N3 and N5.

For example, a score of each node may be determined by scores and edge weights of the neighboring nodes, and scores of all nodes may be recursively determined until convergence is reached. FIG. 5 shows that among three different chemical species H, O, and Cl that constitute one or more chemical reactions, when the species O and Cl have higher scores than that of the species H, the influence of the species O and Cl on the one or more chemical reactions is relatively greater than that of the species H.

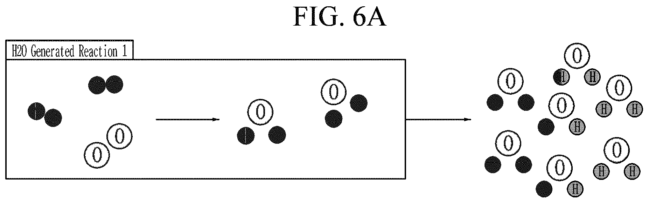

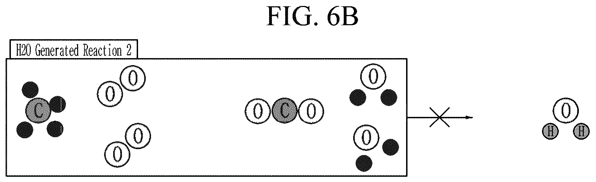

Referring to FIGS. 6A and 6B showing an example of reduction of chemical reactions, as shown in FIG. 6A and FIG. 6B as production reactions for producing $H_2O$, in the case of FIG. 6B, which has a low contribution to the production and destruction of chemical species within a low-dimensional simulation result, even when the production reactions for producing $H_2O$ of FIG. 6B is removed from the original chemical reaction DB, it shows that there is virtually no effect on the density of each species in the steady state.

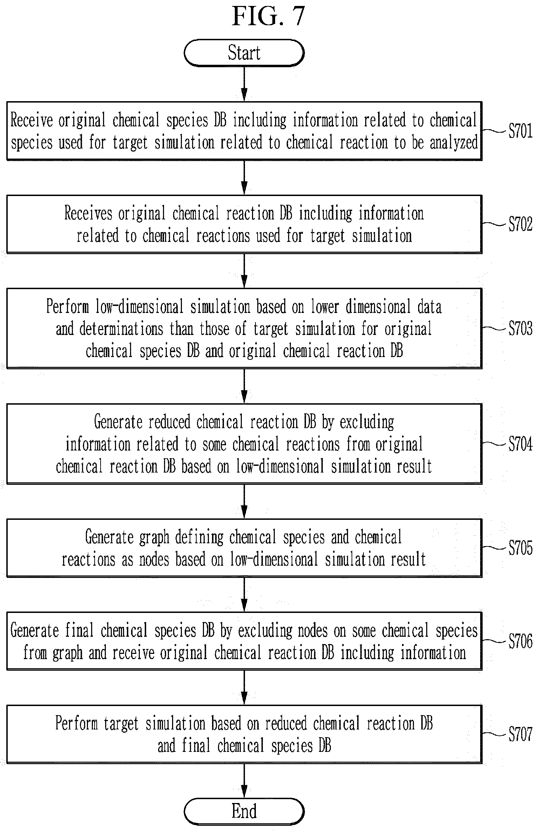

FIG. 7 illustrates a chemical reaction simulation method according to one or more embodiments. Operations S701 to S707 of FIG. 7 may be performed in the sequence and manner as illustrated in FIG. 7. However, one or more of the operations may be performed in a different order, one or more of the operations may be omitted, two or more of the operations may be performed in parallel or simultaneously, and/or other operations may be additionally performed without departing from the scope of the described embodiments.

Referring to FIG. 7, a chemical reaction simulation method according to one or more embodiments may include an operation S701, which receives an original chemical species DB including information related to chemical species used for a target simulation related to a chemical reaction to be analyzed, an operation S702, which receives an original chemical reaction DB including information related to chemical reactions used for target simulation, an operation S703, which performs a low-dimensional simulation based on lower dimensional data and/or determinations than those of the target simulation for the original chemical species DB and the original chemical reaction DB, an operation S704, which generates a reduced chemical reaction DB by excluding information related to one or more chemical reactions from the original chemical reaction DB based on a low-dimensional simulation result, an operation S705, which generates a graph defining chemical species and chemical reactions as nodes based on the low-dimensional simulation result and the reduced chemical reaction DB, an operation S706, which generates a final chemical species DB by excluding nodes on one or more chemical species from the graph, and an operation S707, which performs the target simulation based on the reduced chemical reaction DB and the final chemical species DB.

For more detailed information related to the above method, a reference may be made to the description of the embodiments described in this specification, so redundant description will be omitted herein.

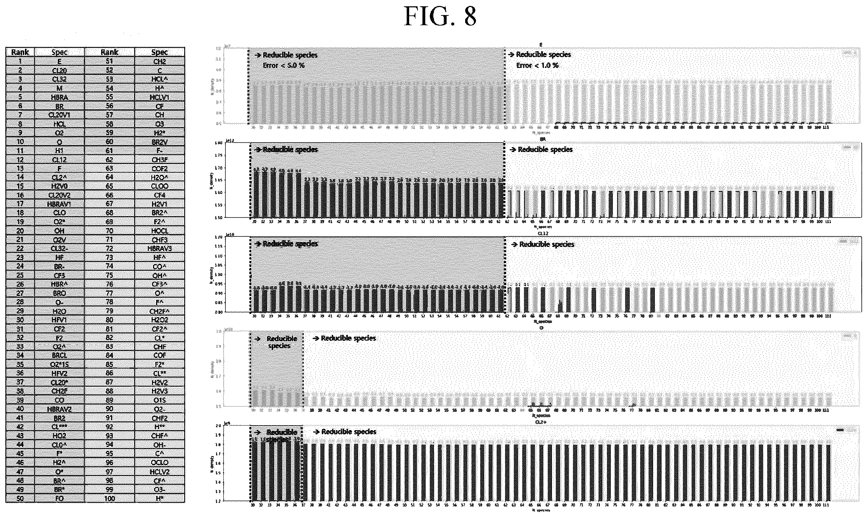

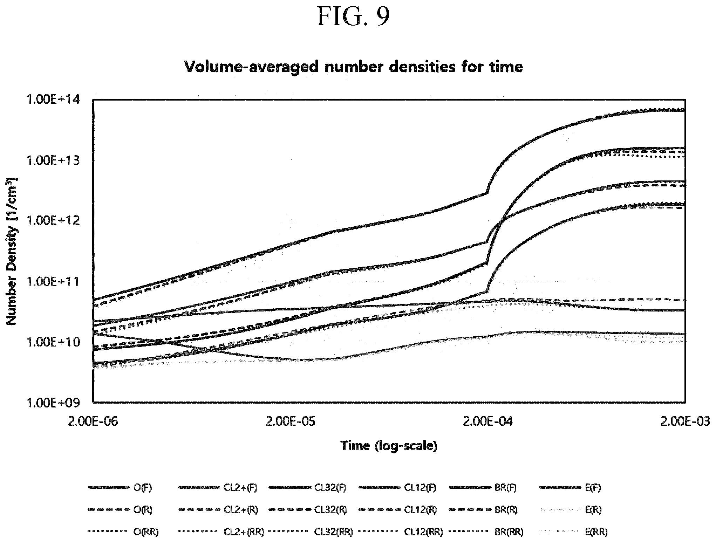

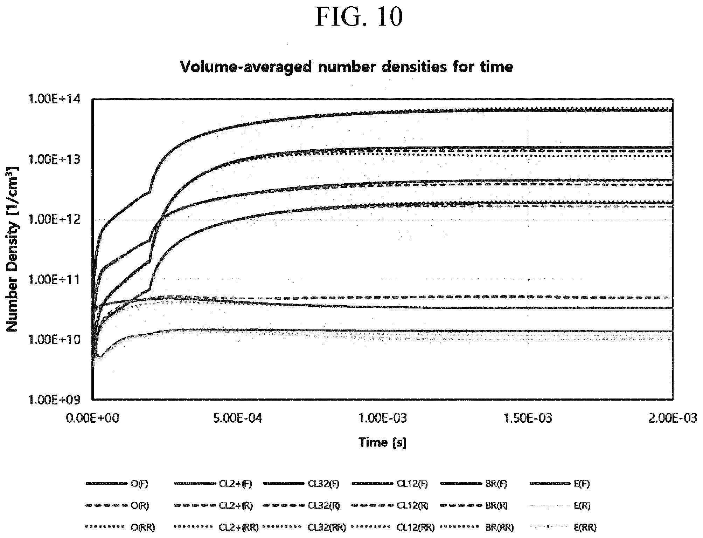

FIG. 8 to FIG. 11 illustrate results of chemical reaction simulations according to one or more embodiments.

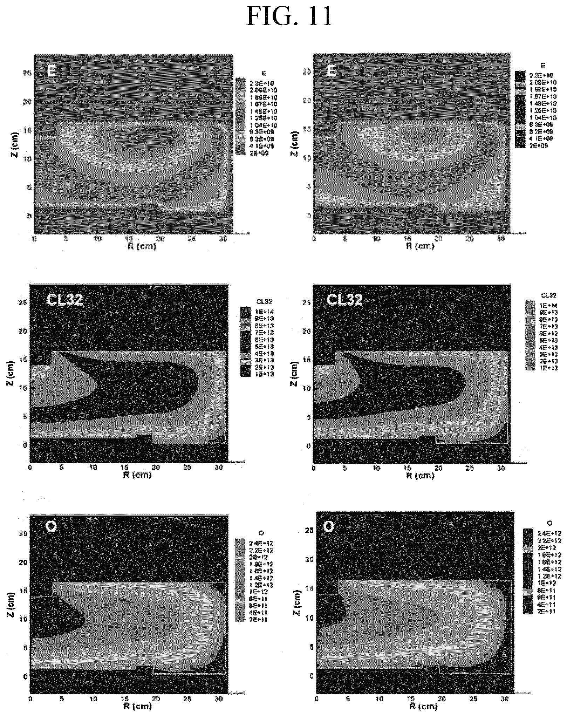

Referring to Figures 8 to 11, to improve the efficiency of plasma etching process analysis, examples were applied, a number density change and a relative error of a reduced set compared to a full set for the species of interest (E, BR, $O_2$, etc.) obtained through low-dimensional simulation are shown, and results of reducing up to 49 chemical species out of the total 111 chemical species are shown. Furthermore, it is shown that when performing high-dimensional simulations based on reduced sets, the chemical reaction simulation device 10 of one or more embodiments may reduce an analysis time while ensuring analysis consistency. In FIG. 11, the three figures on the left correspond to the concentration of three chemicals in the steady state using the high-dimensional simulation, and the three figures on the right correspond to the concentration of these three chemicals in the steady state using the low-dimensional simulation. FIG. 8 shows that '^' indicates a cation state, 'V' indicates a vibrational excited state, and the number following 'V' denotes the order of the vibrational excitation. '*' indicates an electronic excited state, and a greater number of '*' symbols represents a higher energy level of excitation. E represents an electron, CL20 represents the ground state of $Cl_2$, CL32 represents the ground state (2P3/2) of atomic Cl, M represents the total number density of arbitrary molecules, CL12 represents the electronic excited state (2P1/2) of Cl, HCL represents hydrogen chloride (HCl), H1 represents a monoatomic hydrogen molecule (H), BR represents a monoatomic bromine molecule (Br), and O represents a monoatomic oxygen molecule (O). H2V0 represents the ground state of hydrogen molecule ($H_2$), HBRA represents hydrogen bromide (HBr), CL32- represents the anion state of Cl, O2*1S represents the excited state of oxygen molecule ($O_2$), and O1S represents the excited state of atomic oxygen.

High-dimensional simulations may compute a density distribution over time and space of each chemical species that make up a chemical reaction system. Accordingly, by comparing the density distribution of major species over time and space, a degree of preservation of interpretation consistency when applying a reduced set may be confirmed, and a degree of improvement in interpretation efficiency may be confirmed by comparing an interpretation time required. Referring to the volume-averaged number density of the major chemical species over time, results show a same density change over time between a analysis result with all 111 chemical species (full set) as input (solid line) and a result with 62 chemical species (reduced set) as input in response to removing 49 chemical species (dashed line). Differences in the volume average number density in the converged state show relative errors of less than about 30%, such as 0.3% for CL32, 12.4% for O, and 24.8% for E. On the other hand, an interpretation time was reduced by about 53.3% from 56.3 hours (full set) to 26.25 hours (reduced set), improving interpretation efficiency by more than twice. Finally, when referring to the spatial density distribution of the major chemical species in a state where the interpretation has converged, it may be confirmed that both a position of the maximum density and the spatial distribution are similar for both the full set and the reduced set, and an effect according to the examples may be confirmed.

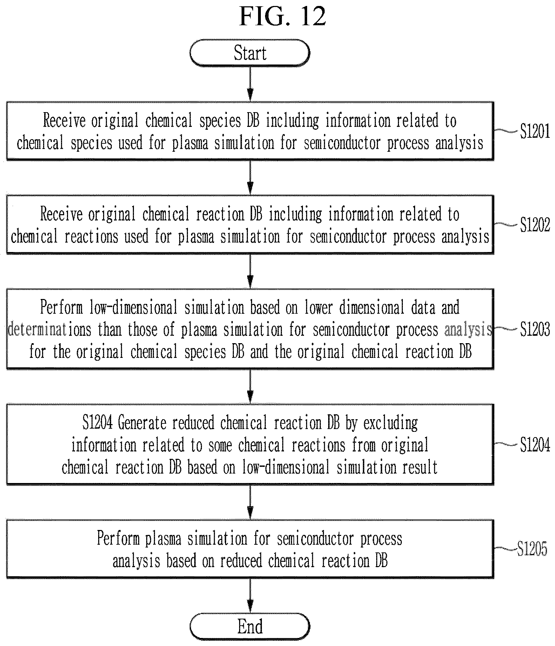

FIG. 12 illustrates a chemical reaction simulation method according to one or more embodiments. Operations S1201 to S1205 of FIG. 12 may be performed in the sequence and manner as illustrated in FIG. 12. However, one or more of the operations may be performed in a different order, one or more of the operations may be omitted, two or more of the operations may be performed in parallel or simultaneously, and/or other operations may be additionally performed without departing from the scope of the described embodiments.

Referring to FIG. 12, a chemical reaction simulation method according to one or more embodiments may include an operation S1201 for receiving an original chemical species DB including information related to chemical species used for a plasma simulation for semiconductor process analysis, an operation S1202 for receiving an original chemical reaction DB including information related to chemical reactions used for the plasma simulation for the semiconductor process analysis, an operation S1203 for performing a low-dimensional simulation based on lower dimensional data and/or determinations than those of the plasma simulation for the semiconductor process analysis for the original chemical species DB and the original chemical reaction DB, an operation S1204 for generating a reduced chemical reaction DB by excluding information related to one or more chemical reactions from the original chemical reaction DB based on a low-dimensional simulation result, and an operation S1205 for performing the plasma simulation for the semiconductor process analysis based on the reduced chemical reaction DB.

For more detailed information related to the above method, a reference may be made to the description of the embodiments described in this specification, so redundant description will be omitted herein.

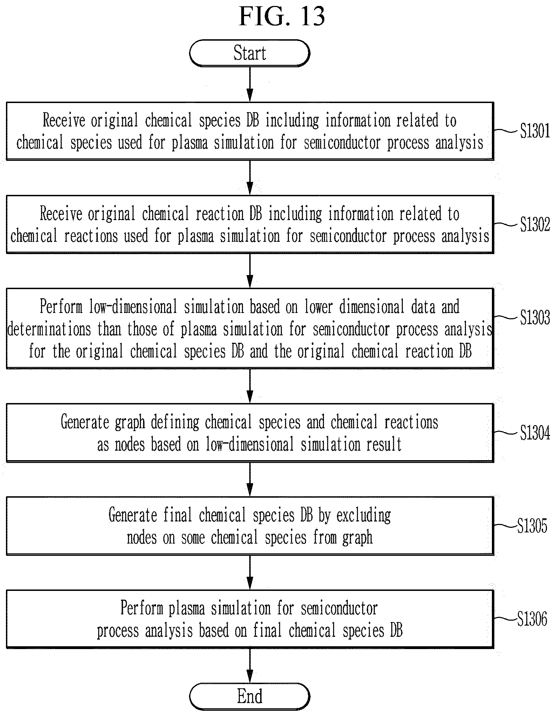

FIG. 13 illustrates a chemical reaction simulation method according to one or more embodiments. Operations S1301 to S1306 of FIG. 13 may be performed in the sequence and manner as illustrated in FIG. 13. However, one or more of the operations may be performed in a different order, one or more of the operations may be omitted, two or more of the operations may be performed in parallel or simultaneously, and/or other operations may be additionally performed without departing from the scope of the described embodiments.

Referring to FIG. 13, a chemical reaction simulation method according to one or more embodiments may include operations S1301, S1302, S1303, S1304, S1305, and S1306.

For more detailed information related to the above method, a reference may be made to the description of the embodiments described in this specification, so redundant description will be omitted herein.

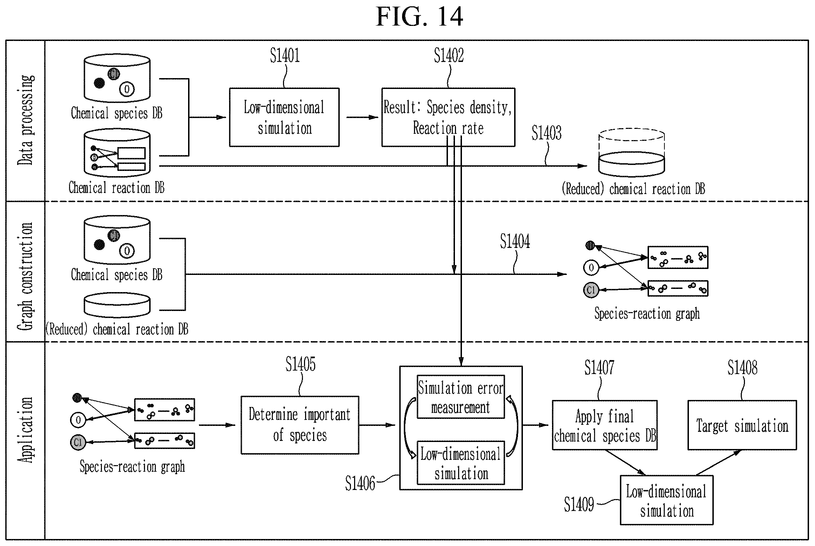

FIG. 14 illustrate a view for describing specific operations of a chemical reaction simulation according to one or more embodiments. Operations S1401 to S1409 of FIG. 14 may be performed in the sequence and manner as illustrated in FIG. 14. However, one or more of the operations may be performed in a different order, one or more of the operations may be omitted, two or more of the operations may be performed in parallel or simultaneously, and/or other operations may be additionally performed without departing from the scope of the described embodiments.

Referring to FIG. 14, the low-dimensional simulation may be performed in the preceding embodiments, so a time to reach a steady state may be drastically reduced by setting a result as an initial condition of a high-dimensional target simulation. Even when using a time-dependent simulation methodology, this method may be considered when the steady state after sufficient time has passed is of greater interest than a progress of the process. The simulation may be conducted with the steady state as a target, so the simulation time may decrease as the initial condition gets closer to the steady state. For example, when an approximate steady-state density is found through a low-dimensional simulation and used as the initial condition for a high-dimensional target simulation, the simulation time may be drastically reduced.

The description of operations S1401 to S1408 may refer to operations S201 to S208 described above with respect to FIG. 2, and in the present embodiment, in operation S1408, before the target simulation is performed based on the reduced chemical reaction DB and the final chemical species DB, an approximation of the steady-state density determined by the low-dimensional simulation in operation S1409 may be provided to the target simulation, and in operation 1408, the target simulation may be performed based on the reduced chemical reaction DB, the final chemical species DB, and the approximation of the steady-state density.

FIG. 15 illustrates a view for describing a computing device according to one or more embodiments.

Referring to FIG. 15, the chemical reaction simulation method and device according to embodiments may be implemented using the computing device 50.

The computing device 50 may include a processor 510 (e.g., one or more processors), a memory 530 (e.g., one or more memories), a user interface input device 540, a user interface output device 550, and a storage device 560 which communicate each other via a bus 520. The computing device 50 may also include a network interface 570 electrically connected to a network 40. The network interface 570 may transmit or receive signals to or from other elements through the network 40.

The processor 510 may be implemented in various types such as a micro controller unit (MCU), an application processor (AP), a central processing unit (CPU), a graphic processing unit (GPU), a neural processing unit (NPU), a quantum processing unit (QPU), etc., and may be any semiconductor device that executes instructions stored in the memory 530 or the storage device 560. The processor 510 may be configured to implement the functions and methods described above with respect to FIGS. 1 to 14. For example, the memory 530 may be or include a non-transitory computer-readable storage medium storing instructions that, when executed by the processor 510, configure the processor 510 to perform any one, any combination, or all of the operations and/or method described herein with reference to FIGS. 1-15.

The memory 530 and the storage device 560 may include various types of volatile or nonvolatile storage media. For example, the memory may include a read only memory (ROM) 531 and a random access memory (RAM) 532. In one or more embodiments, the memory 530 may be positioned internally or externally to the processor 510, and the memory 530 may be connected to the processor 510 through a variety of known means.

In one or more embodiments, one or more of the components or functions of the chemical reaction simulation methods and devices according to the embodiments described above may be implemented as hardware implementing a program or software running on the computing device 50, and the program or software may be stored on a computer-readable medium. For example, a computer-readable medium according to one or more embodiments may be a computer having recorded thereon a program for causing a computer including the processor 510 to execute a program or instructions stored in the memory 530 or a storage device 560 to execute operations included in the implementation of the chemical reaction simulation method and device according to the embodiments.

In one or more embodiments, one or more of components or functions of the chemical reaction simulation methods and devices according to the embodiments may be implemented using hardware or circuitry of the computing device 50, or may be implemented as separate hardware or circuitry that may be electrically connected to the computing device 50.

According to the embodiments, a simulation analysis time may be significantly shortened while securing analysis synthesis by extracting chemical species and reactions required for simulation consistency.

The chemical reaction simulation device 10, DB providing module 101, low-dimensional simulation performing module 102, reduced chemical reaction DB generation module 103, graph generation module 104, final chemical species DB generation module 105, target simulation performing module 106, computing device 50, processor 510, bus 520, memory 530, user interface input device 540, user interface output device 550, storage device 560, network interface 570, network 40, which are described via the descriptions herein, including descriptions with respect to FIGS. 1-15, are implemented by or representative of hardware components. As described above, or in addition to the descriptions above, examples of hardware components that may be used to perform the operations described in this application where appropriate include controllers, sensors, generators, drivers, memories, comparators, arithmetic logic units, adders, subtractors, multipliers, dividers, integrators, and any other electronic components configured to perform the operations described in this application. In other examples, one or more of the hardware components that perform the operations described in this application are implemented by computing hardware, for example, by one or more processors or computers. A processor or computer may be implemented by one or more processing elements, such as an array of logic gates, a controller and an arithmetic logic unit (ALU), a digital signal processor (DSP), a microcomputer, a programmable logic controller, a field-programmable gate array (FPGA), a programmable logic array (PLA), a microprocessor, or any other device or combination of devices that is configured to respond to and execute instructions (e.g., code or coding) in a defined manner to achieve a desired result. In one example, a processor or computer includes, or is connected to, one or more memories storing the instructions or software that are executed by the processor or computer. Hardware components implemented by a processor or computer may execute the instructions or software, such as an operating system (OS) and one or more software applications that run on the OS, to perform the operations described in this application. The hardware components may also access, manipulate, process, create, and store data in response to execution of the instructions or software. For simplicity, the singular term "processor" or "computer" may be used in the description of the examples described in this application, but in other examples multiple processors or computers may be used, or a processor or computer may include multiple processing elements, or multiple types of processing elements, or both, and thus while some references may be made to a singular processor or computer, such references also are intended to refer to multiple processors or computers. For example, a single hardware component or two or more hardware components may be implemented by a single processor, or two or more processors, or a processor and a controller. One or more hardware components may be implemented by one or more processors, or a processor and a controller, and one or more other hardware components may be implemented by one or more other processors, or another processor and another controller. One or more processors, or a processor and a controller, may implement a single hardware component, or two or more hardware components. As described above, or in addition to the descriptions above, example hardware components may have any one or more of different processing configurations, examples of which include a single processor, independent processors, parallel processors, single-instruction single-data (SISD) multiprocessing, single-instruction multiple-data (SIMD) multiprocessing, multiple-instruction single-data (MISD) multiprocessing, and multiple-instruction multiple-data (MIMD) multiprocessing. Thus, references to a processor herein mean processing circuitry (e.g., circuitry that includes one or more processing element(s) circuits). One or more processors comprising processing circuitry also refers to each processor comprising processing circuitry, as well as some or all of the one or more processors comprising the same processing circuitry. In addition, processors(s) and controller(s), as a non-limiting example, do not mean human processing or human control, but rather, refer to hardware components as described herein, as non-limiting examples.

The methods illustrated in, and discussed with respect to, FIGS. 1-15 that perform the operations described in this application are performed by computing hardware, for example, by one or more processors or computers, implemented as described above implementing the instructions (e.g., computer or processor/processing device readable instructions) or software to perform the operations described in this application that are performed by the methods. For example, a single operation or two or more operations may be performed by a single processor, or two or more processors, or a processor and a controller. One or more operations may be performed by one or more processors, or a processor and a controller, and one or more other operations may be performed by one or more other processors, or another processor and another controller. One or more processors, or a processor and a controller, may perform a single operation, or two or more operations. References to a processor, or one or more processors, as a non-limiting example, configured to perform two or more operations refers to a processor or two or more processors being configured to collectively perform all of the two or more operations, as well as a configuration with the two or more processors respectively performing any corresponding one of the two or more operations (e.g., with a respective one or more processors being configured to perform each of the two or more operations, or any respective combination of one or more processors being configured to perform any respective combination of the two or more operations). Likewise, a reference to a processor-implemented method is a reference to a method that is performed by one or more processors or other processing or computing hardware of a device or system.

The instructions or software to control computing hardware, for example, one or more processors or computers, to implement the hardware components and perform the methods as described above may be written as computer programs, code segments, or other executable instructions or any combination thereof, for individually or collectively instructing or configuring the one or more processors or computers to operate as a machine or special-purpose computer to perform the operations that are performed by the hardware components and the methods as described above. In one example, the instructions or software include machine code that is directly executed by the one or more processors or computers, such as machine code produced by a compiler. In another example, the instructions or software includes higher-level code that is executed by the one or more processors or computer using an interpreter. The instructions or software may be written using any programming language based on the block diagrams and the flow charts illustrated in the drawings and the corresponding descriptions herein, which disclose algorithms for performing the operations that are performed by the hardware components and the methods as described above.

The instructions or software to control computing hardware, for example, one or more processors or computers, to implement the hardware components and perform the methods as described above, and any associated data, data files, and data structures, may be recorded, stored, or fixed in or on one or more non-transitory computer-readable storage media, and thus, not a signal per se. Thus, references herein to storage media mean storage media hardware, and does not mean transitory media, nor a signal per se. As described above, or in addition to the descriptions above, examples of a non-transitory computer-readable storage medium include one or more of any of read-only memory (ROM), random-access programmable read only memory (PROM), electrically erasable programmable read-only memory (EEPROM), random-access memory (RAM), dynamic random access memory (DRAM), static random access memory (SRAM), flash memory, non-volatile memory, CD-ROMs, CD-Rs, CD+Rs, CD-RWs, CD+RWs, DVD-ROMs, DVD- Rs, DVD+Rs, DVD-RWs, DVD+RWs, DVD-RAMs, BD-ROMs, BD-Rs, BD-R LTHs, BD-REs, blue-ray or optical disk storage, hard disk drive (HDD), solid state drive (SSD), flash memory, a card type memory such as a multimedia card or a micro card (for example, secure digital (SD) or extreme digital (XD)), magnetic tapes, floppy disks, magneto-optical data storage devices, optical data storage devices, hard disks, solid-state disks , and/or any other device that is configured to store the instructions or software and any associated data, data files, and data structures in a non-transitory manner and provide the instructions or software and any associated data, data files, and data structures to one or more processors or computers so that the one or more processors or computers can execute the instructions. In one example, the instructions or software and any associated data, data files, and data structures are distributed over network-coupled computer systems so that the instructions and software and any associated data, data files, and data structures are stored, accessed, and executed in a distributed fashion by the one or more processors or computers.

While this disclosure includes specific examples, it will be apparent after an understanding of the disclosure of this application that various changes in form and details may be made in these examples without departing from the scope of the claims and their equivalents. The examples described herein are to be considered in a descriptive sense only, and not for purposes of limitation. Descriptions of features or aspects in each example are to be considered as being applicable to similar features or aspects in other examples. Suitable results may be achieved if the described techniques are performed in a different order, and/or if components in a described system, architecture, device, or circuit are combined in a different manner, and/or replaced or supplemented by other components or their equivalents.

Embodiments of the present application are also defined in the following clauses:

Clause 1. A processor-implemented method comprising:

performing, based on an original chemical species database (DB) and an original chemical reaction DB, a low dimensional simulation based on lower dimensional data and determinations than those of a target simulation corresponding to a chemical reaction to be analyzed, the original chemical species DB comprising information corresponding to a chemical species used for the target simulation, and the original chemical reaction DB comprising information corresponding to a chemical reaction used for the target simulation; generating a reduced chemical reaction DB by excluding information corresponding to one or more chemical reactions from the original chemical reaction DB based on a result of the low-dimensional simulation; and performing the target simulation based on the reduced chemical reaction DB and the final chemical species DB.

Clause 2. The method of clause 1, wherein the generating of the reduced chemical reaction DB comprises:

extracting information corresponding to a chemical species density and a reaction rate from the result of the low-dimensional simulation; and

determining contribution to generation and consumption of chemical species for all chemical reactions associated with the original chemical reaction DB based on the information corresponding to the density and the reaction rate of the chemical species, and generating a reduced chemical reaction DB by excluding information corresponding to one or more chemical reactions selected based on the contribution from the original chemical reaction DB.

Clause 3. The method of clause 2, wherein the generating of the reduced chemical reaction DB comprises:

determining a first contribution to production and consumption of a first chemical species for all chemical reactions corresponding to the first chemical species corresponding to the original chemical reaction DB;

registering chemical reactions whose first contribution exceeds a predetermined first threshold among all chemical reactions in the original chemical reaction DB in a white list; and

generating the reduced chemical reaction DB by excluding information corresponding to one or more chemical reactions that are not registered in the white list from the original chemical reaction DB.

Clause 4. The method of clause 3, wherein the generating of the reduced chemical reaction DB further comprises:

determining a second contribution to production and consumption of a second chemical species for all chemical reactions of the second chemical species corresponding to the original chemical reaction DB and different from the first chemical species; and

registering a chemical reaction having the second contribution that exceeds the first threshold, among all chemical reactions of the original chemical reaction DB, in the white list.

Clause 5. The method of clause 1, further comprising:

generating a graph defining the chemical species and the chemical reaction as nodes based on the result of the low-dimensional simulation and the reduced chemical reaction DB; and

generating a final chemical species DB by excluding nodes corresponding to one or more chemical species from the graph,

wherein the performing of the target simulation comprises performing the target simulation based on the final chemical species DB.

Clause 6. The method of clause 5, wherein the generating of the graph comprises:

forming an edge between a node representing a chemical species and a node representing a chemical reaction based on the reduced chemical reaction DB, depending on whether a chemical reaction is involved; and

setting a value of a reaction rate as an edge weight based on a result of the low-dimensional simulation.

Clause 7. The method of clause 6, wherein the generating of the graph comprises generating an undirected species-reaction bipartite graph as a graph using the node, the edge, and the edge weight.

Clause 8. The method of clause 5, wherein the generating of the final chemical species DB comprises:

determining an importance score for all nodes in the graph;

determining an importance ranking for all chemical species by considering the scores for the nodes representing chemical species in the graph; and

generating the final chemical species DB based on the importance ranking.

Clause 9. The method of clause 8, wherein the generating of the final chemical species DB comprises:

determining a reduced set formed of a predetermined number of chemical species selected in order of increasing importance;

determining a full set formed of all chemical species represented in the graph;

determining a relative error between a result of the low-dimensional simulation performed on the reduced set and a result of the low-dimensional simulation performed on the full set; and

generating the reduced set having the relative error less than or equal to a predetermined second threshold as the final chemical species DB.

Clause 10. The method of clause 9,

wherein the relative error is determined according to a following Equation:

$$\text{RE} = \left( \frac{n_R - n_F}{n_F} \right) \times 100$$

wherein RE indicates the relative error, $n_R$ indicates a number density of the species of interest based on the reduced set, and $n_F$ indicates a number density of the species of interest based on the full set.

Clause 11. The method of clause 1, wherein the target simulation comprises a low-temperature plasma simulation for semiconductor process analysis.

Clause 12. An electronic device comprising:

one or more processors comprising processing circuitry; and
memory comprising one or more storage media storing instructions that, when executed individually or collectively by the one or more processors, cause the electronic device to:

perform, based on an original chemical species database (DB) and an original chemical reaction DB, a low dimensional simulation based on lower dimensional data and determinations than those of a target simulation corresponding to a chemical reaction to be analyzed, the original chemical species DB comprising information corresponding to a chemical species used for the target simulation, and the original chemical reaction DB comprising information corresponding to a chemical reaction used for the target simulation; generate a reduced chemical reaction DB by excluding information corresponding to one or more chemical reactions from the original chemical reaction DB based on a result of the low-dimensional simulation; and perform the target simulation based on the reduced chemical reaction DB.

Clause 13. The electronic device of clause 12, wherein, for the generating of the reduced chemical reaction DB, the execution of the instructions causes the electronic device to:

extract information corresponding to a chemical species density and a reaction rate from the result of the low-dimensional simulation; and
determine contribution to generation and consumption of chemical species for all chemical reactions associated with the original chemical reaction DB based on the information corresponding to the density and the reaction rate of the chemical species, and generating a reduced chemical reaction DB by excluding information corresponding to one or more chemical reactions selected based on the contribution from the original chemical reaction DB.

Clause 14. The electronic device of clause 13, wherein, for the generating of the reduced chemical reaction DB, the execution of the instructions causes the electronic device to:

determine a first contribution to production and consumption of a first chemical species for all chemical reactions corresponding to the first chemical species corresponding to the original chemical reaction DB;
register chemical reactions whose first contribution exceeds a predetermined first threshold among all chemical reactions in the original chemical reaction DB in a white list; and
generate the reduced chemical reaction DB by excluding information corresponding to one or more chemical reactions that are not registered in the white list from the original chemical reaction DB.

Clause 15. A processor-implemented method comprising:

performing, based on an original chemical species database (DB) and an original chemical reaction DB, a low dimensional simulation based on lower dimensional data and determinations than those of a target simulation corresponding to a chemical reaction to be analyzed, the original chemical species DB comprising information corresponding to a chemical species used for the target simulation, and the original chemical reaction DB comprising information corresponding to a chemical reaction used for the target simulation;
generating a graph defining the chemical species and the chemical reaction as nodes based on a result of the low-dimensional simulation;
generating a final chemical species DB by excluding nodes corresponding to one or more chemical species from the graph; and
performing the target simulation based on the final chemical species DB.

Clause 16. The method of clause 15, wherein the generating of the graph comprises:

forming an edge between a node representing a chemical species and a node representing a chemical reaction depending on whether a chemical reaction is involved; and

setting a value of a reaction rate as an edge weight based on a result of the low-dimensional simulation.

Clause 17. The method of clause 16, wherein the generating of the graph comprises generating an undirected species-reaction bipartite graph as a graph using the node, the edge, and the edge weight.

Clause 18. The method of clause 15, wherein the generating of the final chemical species DB comprises:

determining an importance score for all nodes in the graph;
determining an importance ranking for all chemical species by considering the scores for the nodes representing chemical species in the graph; and
generating the final chemical species DB based on the importance ranking.

Clause 19. The method of clause 18, wherein the generating of the final chemical species DB comprises:

determining a reduced set formed of a predetermined number of chemical species selected in order of increasing importance;
determining a full set formed of all chemical species represented in the graph;
determining a relative error between a result of the low-dimensional simulation performed on the reduced set and a result of the low-dimensional simulation performed on the full set; and
generating the reduced set having the relative error less than or equal to a predetermined second threshold as the final chemical species DB.

Clause 20. The method of clause 19,

wherein the relative error is determined according to a following Equation:

$$\mathrm{RE} = \left(\frac{n_R - n_F}{n_F}\right) \times 100$$

wherein RE indicates the relative error, $n_R$ indicates a number density of the species of interest based on the reduced set, and $n_F$ indicates a number density of the species of interest based on the full set.

Therefore, in addition to the above and all drawing disclosures, the scope of the disclosure is also inclusive of the claims and their equivalents, i.e., all variations within the scope of the claims and their equivalents are to be construed as being included in the disclosure.

## Claims

1. A processor-implemented method comprising:

performing, based on an original chemical species database, DB, and an original chemical reaction DB, a low-dimensional simulation based on lower dimensional data and/or determinations than those of a target simulation corresponding to a chemical reaction to be analyzed, the original chemical species DB comprising information corresponding to a chemical species used for the target simulation, and the original chemical reaction DB comprising information corresponding to a chemical reaction used for the target simulation;
generating a reduced chemical reaction DB by excluding information corresponding to one or more chemical reactions from the original chemical reaction DB based on a result of the low-dimensional simulation;
performing the target simulation based on the reduced chemical reaction DB and a final chemical species DB;
controlling at least one parameter of a chemical reaction based on the target simulation.

2. The method of claim 1, wherein the generating of the reduced chemical reaction DB comprises:

extracting information corresponding to a chemical species density and a reaction rate from the result of the low-dimensional simulation; and
determining contribution to generation and/or consumption of chemical species for all chemical reactions associated with the original chemical reaction DB based on the information corresponding to the density and the reaction rate of the chemical species, and generating a reduced chemical reaction DB by excluding information corresponding to one or more chemical reactions selected based on the contribution from the original chemical reaction DB.

3. The method of claim 2, wherein the generating of the reduced chemical reaction DB comprises:

determining a first contribution to production and/or consumption of a first chemical species for all chemical reactions of the first chemical species corresponding to the original chemical reaction DB;
registering a chemical reaction whose first contribution exceeds a predetermined first threshold among all chemical reactions in the original chemical reaction DB in a white list; and
generating the reduced chemical reaction DB by excluding information corresponding to one or more chemical reactions that are not registered in the white list from the original chemical reaction DB.

4. The method of claim 3, wherein the generating of the reduced chemical reaction DB further comprises:

determining a second contribution to production and/or consumption of a second chemical species for all chemical reactions of the second chemical species corresponding to the original chemical reaction DB and different from the first chemical species; and
registering a chemical reaction having the second contribution that exceeds the first threshold, among all chemical reactions of the original chemical reaction DB, in the white list.

5. The method of any of the previous claims, further comprising:

generating a graph defining the chemical species and the chemical reaction as nodes based on the result of the low-dimensional simulation and the reduced chemical reaction DB; and
generating the final chemical species DB by excluding nodes corresponding to one or more chemical species from the graph,
wherein the performing of the target simulation comprises performing the target simulation based on the final chemical species DB.

6. The method of claims 2 and 5, wherein the generating of the graph comprises:

forming an edge between a node representing a chemical species and a node representing a chemical reaction based on the reduced chemical reaction DB, depending on whether a chemical reaction is involved; and
setting a value of a reaction rate as an edge weight based on a result of the low-dimensional simulation.

7. The method of claim 6, wherein the generating of the graph comprises generating an undirected species-reaction bipartite graph as a graph using the node, the edge, and the edge weight.

8. The method of any of claims 5 - 7, wherein the generating of the final chemical species DB comprises:

determining an importance score for all nodes in the graph;
determining an importance ranking for all chemical species by considering the importance scores for the nodes representing chemical species in the graph; and
generating the final chemical species DB based on the importance ranking.

9. The method of any of claims 5 - 8, wherein the generating of the final chemical species DB comprises:

determining a reduced set formed of a predetermined number of chemical species selected in order of increasing importance;
determining a full set formed of all chemical species represented in the graph;
determining a relative error between a result of the low-dimensional simulation performed on the reduced set and a result of the low-dimensional simulation performed on the full set; and
generating the reduced set having the relative error less than or equal to a predetermined second threshold as the final chemical species DB.

10. The method of claim 9,

wherein the relative error is determined according to a following Equation:

$$RE = \left(\frac{n_R - n_F}{n_F}\right) \times 100$$

wherein RE indicates the relative error, $n_R$ indicates a number density of the species of interest based on the reduced set, and $n_F$ indicates a number density of the species of interest based on the full set.

11. The method of any of the previous claims, wherein the target simulation comprises a low-temperature plasma simulation for semiconductor process analysis.

12. An electronic device comprising:

one or more processors comprising processing circuitry; and
memory comprising one or more storage media storing instructions that, when executed individually or collectively by the one or more processors, cause the electronic device to:
perform, based on an original chemical species database, DB, and an original chemical reaction DB, a low-dimensional simulation based on lower dimensional data and/or determinations than those of a target simulation corresponding to a chemical reaction to be analyzed, the original chemical species DB comprising information corresponding to a chemical species used for the target simulation, and the original chemical reaction DB comprising information corresponding to a chemical reaction used for the target simulation;
generate a reduced chemical reaction DB by excluding information corresponding to one or more chemical reactions from the original chemical reaction DB based on a result of the low-dimensional simulation;
perform the target simulation based on the reduced chemical reaction DB;
control at least one parameter of a chemical reaction based on the target simulation.

13. The electronic device of claim 12, wherein, for the generating of the reduced chemical reaction DB, the execution of the instructions causes the electronic device to:

extract information corresponding to a chemical species density and a reaction rate from the result of the low-dimensional simulation; and
determine contribution to generation and/or consumption of chemical species for all chemical reactions associated with the original chemical reaction DB based on the information corresponding to the density and the reaction rate of the chemical species, and generating a reduced chemical reaction DB by excluding information corresponding to one or more chemical reactions selected based on the contribution from the original chemical reaction DB.

14. The electronic device of claim 13, wherein, for the generating of the reduced chemical reaction DB, the execution of the instructions causes the electronic device to:

determine a first contribution to production and/or consumption of a first chemical species for all chemical reactions of the first chemical species corresponding to the original chemical reaction DB;
register a chemical reaction whose first contribution exceeds a predetermined first threshold among all chemical reactions in the original chemical reaction DB in a white list; and
generate the reduced chemical reaction DB by excluding information corresponding to one or more chemical reactions that are not registered in the white list from the original chemical reaction DB.

15. The Electronic device of any of claims 12 - 14, wherein, for the generating of the reduced chemical reaction DB, the execution of the instructions causes the electronic device to:

generate a graph defining the chemical species and the chemical reaction as nodes based on the result of the low-dimensional simulation and the reduced chemical reaction DB; and
generate the final chemical species DB by excluding nodes corresponding to one or more chemical species from the graph,
wherein the performing of the target simulation comprises performing the target simulation based on the final chemical species DB.

# FIG. 1

10

| | |
|---|---|
| DB providing module (101) | low-dimensional simulation performing module (102) |
| reduced chemical reaction DB generation module (103) | graph generation module (104) |
| final chemical species DB generation module (105) | target simulation performing module (106) |

# FIG. 2

**Data processing**

Chemical species DB
Chemical reaction DB

S201 — Low-dimensional simulation

S202 — Result: Species density, Reaction rate

S203 — (Reduced) chemical reaction DB

**Graph construction**

Chemical species DB
(Reduced) chemical reaction DB

S204 — Species-reaction graph

**Application**

Species-reaction graph

S205 — Determine important of species

S206 — Simulation error measurement / Low-dimensional simulation

S207 — Apply final chemical species DB

S208 — Target simulation

EP 4 693 302 A1

## FIG. 3A

# FIG. 3B

FIG. 4

FIG. 5

EP 4 693 302 A1

FIG. 6A

27

FIG. 6B

H2O Generated Reaction 2

# FIG. 7

Start

Receive original chemical species DB including information related to chemical species used for target simulation related to chemical reaction to be analyzed ~S701

Receives original chemical reaction DB including information related to chemical reactions used for target simulation ~S702

Perform low-dimensional simulation based on lower dimensional data and determinations than those of target simulation for original chemical species DB and original chemical reaction DB ~S703

Generate reduced chemical reaction DB by excluding information related to some chemical reactions from original chemical reaction DB based on low-dimensional simulation result ~S704

Generate graph defining chemical species and chemical reactions as nodes based on low-dimensional simulation result ~S705

Generate final chemical species DB by excluding nodes on some chemical species from graph and receive original chemical reaction DB including information ~S706

Perform target simulation based on reduced chemical reaction DB and final chemical species DB ~S707

End

FIG. 8

# FIG. 9

**Volume-averaged number densities for time**

FIG. 10

**Volume-averaged number densities for time**

EP 4 693 302 A1

# FIG. 11

# FIG. 12

```
                        ( Start )
                            │
                            ▼
┌──────────────────────────────────────────────────────┐
│  Receive original chemical species DB including       │
│  information related to                                │──  S1201
│ chemical species used for plasma simulation for        │
│ semiconductor process analysis                         │
└──────────────────────────────────────────────────────┘
                            │
                            ▼
┌──────────────────────────────────────────────────────┐
│  Receive original chemical reaction DB including       │
│  information related to                                │──  S1202
│ chemical reactions used for plasma simulation for      │
│ semiconductor process analysis                         │
└──────────────────────────────────────────────────────┘
                            │
                            ▼
┌──────────────────────────────────────────────────────┐
│  Perform low-dimensional simulation based on lower     │
│  dimensional data and                                  │
│ determinations than those of plasma simulation for     │── S1203
│ semiconductor process analysis                         │
│ for the original chemical species DB and the original  │
│ chemical reaction DB                                   │
└──────────────────────────────────────────────────────┘
                            │
                            ▼
┌──────────────────────────────────────────────────────┐
│  S1204 Generate reduced chemical reaction DB by        │
│  excluding                                             │
│  information related to some chemical reactions from    │── S1204
│  original                                              │
│  chemical reaction DB based on low-dimensional         │
│  simulation result                                     │
└──────────────────────────────────────────────────────┘
                            │
                            ▼
┌──────────────────────────────────────────────────────┐
│   Perform plasma simulation for semiconductor process  │── S1205
│   analysis based on reduced chemical reaction DB       │
└──────────────────────────────────────────────────────┘
                            │
                            ▼
                        ( End )
```

# FIG. 13

Start

Receive original chemical species DB including information related to chemical species used for plasma simulation for semiconductor process analysis — S1301

Receive original chemical reaction DB including information related to chemical reactions used for plasma simulation for semiconductor process analysis — S1302

Perform low-dimensional simulation based on lower dimensional data and determinations than those of plasma simulation for semiconductor process analysis for the original chemical species DB and the original chemical reaction DB — S1303

Generate graph defining chemical species and chemical reactions as nodes based on low-dimensional simulation result — S1304

Generate final chemical species DB by excluding nodes on some chemical species from graph — S1305

Perform plasma simulation for semiconductor process analysis based on final chemical species DB — S1306

End

## FIG. 14

**Data processing**

Chemical species DB

Chemical reaction DB

S1401 — Low-dimensional simulation

S1402 — Result: Species density, Reaction rate

S1403 — (Reduced) chemical reaction DB

**Graph construction**

Chemical species DB

(Reduced) chemical reaction DB

S1404

Species-reaction graph

**Application**

Species-reaction graph

S1405 — Determine important of species

Simulation error measurement

S1406 — Low-dimensional simulation

S1407 — Apply final chemical species DB

S1408 — Target simulation

S1409 — Low-dimensional simulation

EP 4 693 302 A1

# FIG. 15

# EUROPEAN SEARCH REPORT

**Application Number**

EP 25 19 4642

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | YUDONG SHEN ET AL: "Time-integrated species flux analysis: A novel method for kinetic reduction and pathway analysis in pyrolysis process", AICHE JOURNAL, JOHN WILEY & SONS, INC, US, vol. 70, no. 10, 19 July 2024 (2024-07-19), page n/a, XP072724806, ISSN: 0001-1541, DOI: 10.1002/AIC.18532 * the whole document * | 1-15 | INV. G16C20/10 |
| X | HARIRCHI FARSHAD ET AL: "On sparse identification of complex dynamical systems: A study on discovering influential reactions in chemical reaction networks", FUEL, IPC SIENCE AND TECHNOLOGY PRESS , GUILDFORD, GB, vol. 279, 22 June 2020 (2020-06-22), XP086241995, ISSN: 0016-2361, DOI: 10.1016/J.FUEL.2020.118204 [retrieved on 2020-06-22] * the whole document * | 1-15 | |
| A | SUN BOWEN ET AL: "Simplification of plasma chemistry by means of vital nodes identification", JOURNAL OF APPLIED PHYSICS, AMERICAN INSTITUTE OF PHYSICS, 2 HUNTINGTON QUADRANGLE, MELVILLE, NY 11747, vol. 130, no. 9, 3 September 2021 (2021-09-03), XP012259329, ISSN: 0021-8979, DOI: 10.1063/5.0063068 [retrieved on 2021-09-03] * the whole document * | 1-15 | **TECHNICAL FIELDS SEARCHED (IPC)**<br><br>G16C |

The present search report has been drawn up for all claims

| Place of search | Date of completion of the search | Examiner |
|---|---|---|
| The Hague | 8 December 2025 | Denoual, Matthieu |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)